(19) Europäisches Patentamt
European Patent Office
Office européen des brevets

(11) Publication number: **0 141 884**
**B1**

(12) # EUROPEAN PATENT SPECIFICATION

(45) Date of publication of patent specification: **20.07.88**

(51) Int. Cl.$^4$: **C 07 D 295/08,** A 61 K 31/495

(21) Application number: **83306814.1**

(22) Date of filing: **09.11.83**

(54) **Novel phenylpiperazine derivatives, process for preparing them and therapeutic compositions containing them.**

(43) Date of publication of application:
**22.05.85 Bulletin 85/21**

(45) Publication of the grant of the patent:
**20.07.88 Bulletin 88/29**

(84) Designated Contracting States:
**DE FR GB IT**

(56) References cited:
**ES-A- 515 541**
**GB-A-1 437 868**
**GB-A-2 027 019**
**US-A-4 150 137**

(73) Proprietor: **JUSTE, S.A. QUIMICO-FARMACEUTICA**
***Julio Camba, 7***
**Madrid (ES)**

(72) Inventor: **Martin Jimenez, Jose Luis**
**39 Las Huertas, Majadahonda**
**Madrid (ES)**
Inventor: **Carretero Colon, Jose Ma.**
**151 Moratalaz Ave.**
**Madrid (ES)**
Inventor: **Alamo Gonzalez, Cecilio**
**8 Gerona, Alcala de Henares**
**Madrid (ES)**

(74) Representative: **Cole, Paul Gilbert et al**
**Hughes Clark Andrews & Byrne**
**63 Lincoln's Inn Fields**
**London WC2A 3JU (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 141 884 B1**

Courier Press, Leamington Spa, England.

**Description**

This invention relates to meta-substituted phenylpiperazine derivatives that have valuable therapeutic properties as non-narcotic analgesics, and to a process for preparing them. It also relates to therapeutic compositions comprising as active ingredient one of these derivatives, for oral, rectal or parenteral administration.

According to Manoury et al., J. Med. Chem. *22,* 5, 554 (1979), the esterification of certain quinoline anthranilic acids (used as antiinflammatory and analgesic agents in clinical practice) with phenylpiperazinyl alkanols gives rise to compounds with increased analgesic activity and reduced antiinflammatory activity, so that they are less ulcerogenic. The compounds are better absorbed by virtue of their increased liposolubility. The compound 2-(4-isobutylphenyl) propionic acid is well known and widely employed in general clinical practice as an antiinflammatory analgesic. In the hope of obtaining compounds with higher analgesic activity and lesser degree of toxicity, we have prepared several derivatives of the 2-(4-isobutylphenyl) butyric acid of the formula

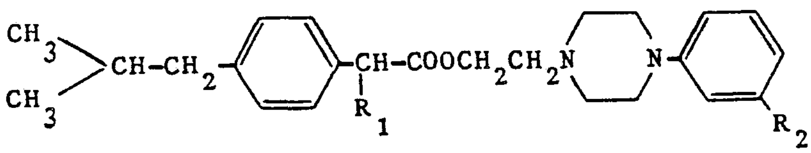

I

wherein $R_1$, is an ethyl group and $R_2$ is a hydrogen or chlorine atom or a trifluoromethyl group (see Spanish Patent No. 515,541). The compounds were found to have interesing analgesic properties and low toxicity.

We have now found that the compounds of general formula:

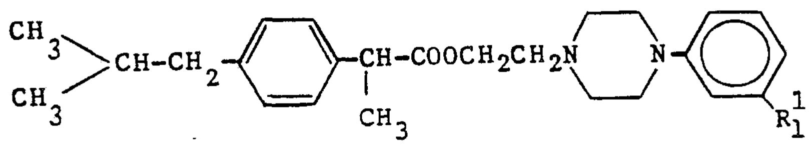

Ia

wherein $R_1$ (equivalent to $R_2$ in Formula I) represents a hydrogen or chlorine atom or a trifluoromethyl group have a therapeutic index (ratio of $LD_{50}/ED_{50}$) higher than those of their individual components and that they have more reduced antiinflammatory properties and better absorption and bioavailability due to their increased liposolubility. The compounds of Formula Ia have been found to have very surprising analgesic properties and pharmacological studies have shown that they do not exhibit the harmful side effects of narcotic type analgesics probably because (so it is believed) their mechanism of action does not involve the same type of receptors.

The compounds of general formula Ia may also be used as medicaments in the form of the free bases as well as under the form of the physiological acceptable salts obtained from the addition of organic or inorganic acids (hydrochloric acid, sulphuric acid, malic acid, succinic acid, citric acid, maleic acid) to the piperazinyl nitrogens.

The invention also provides processes for preparing the general formula Ia. One such process involves esterifying a phenylpiperazinyl ethanol of general formula:

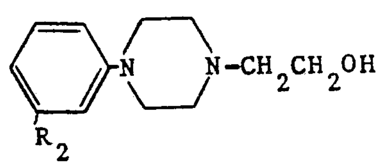

II

wherein $R_2$ represents a hydrogen or chlorine atom or trifluoromethyl group with 2-(4-isobutylphenyl)-propionyl chloride:

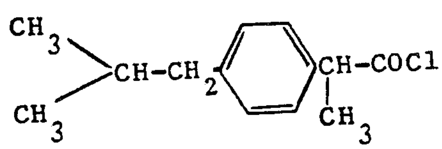

III

The reaction can be carried out at low temperatures (<5°C) and in the presence of a base such as sodium carbonate or pyridine. Compounds of formula II may readily be obtained by reaction of the corresponding phenylpiperazine:

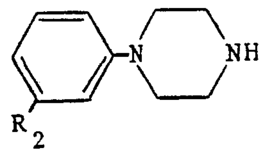

IV

wherein $R_2$ represents a hydrogen or chlorine atom or trifluoromethyl group with 2-chloroethanol. An alternative process for preparing a compound according to the invention involves esterifying 2-chloroethanol with the acid chloride III to give the compound:

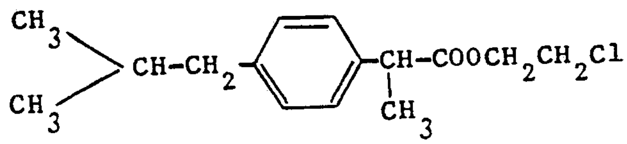

V

which upon subsequent reaction with phenylpiperazine of formula IV in a basic medium yields a compound of formula Ia.

The compounds of the invention can be presented for administration to human beings by the oral, rectal or parenteral routes in the form of the free bases as well as in the form of their physiologically acceptable salts. For oral administration they may be presented in the form of tablets, capsules, granules, sugar coated tablets, suspensions or solutions. These preparations may incorporate acceptable auxiliary agents as talc, magnesium stearate, vegetable gums, sucrose, mannitol, lactose, starch, polyvinylpyrrolidone, a tween surfactant and flavouring and colouring agents. For rectal administration they may be presented as suppositories containing a compound of the invention together with neutral fats such as mono-, di- and triglycerides of fatty acids or polyethyleneglycols. The compounds of the invention may be presented for parenteral use in association with an appropriate hydroalcoholic vehicle and with an antioxidant and heavy metal chelating agents.

The unit dosage presentations may contain 100 to 700 mg of a compound according to the invention as active ingredient. Unit dosage forms of oral administration preferably contain 200 to 500 mg of the compound, those for parenteral administration preferably contain 100 to 200 mg, and suppositories preferably contain 500—700 mg thereof. Such preparations may be made by methods well known in the art, and the active ingredients of the present invention may also be incorporated into the novel composition with other known therapeutically active compounds.

The studies of analgesic activity for these compounds have been carried out over 2,178 mice by using the "writhing test" method. 2-Phenylbenzoquinone was used as nociceptive agent and the material under test was administered by the oral route. In all the cases the $ED_{50}$, $ED_{16}$ and $ED_{84}$ (minimum dose for each product which reduces the writhes by 50, 16 and 84% respectively) were calculated, as well as the 95% confidence limits for the $ED_{50}$. The oral acute toxicity study ($LD_{50}$ determination) has been performed using 770 Swiss mice (half males and half females) weighing 18—22 g.

A. Analgesic activity ($ED_{50}$):

A slight modification of the method described by Hendershot and Forshait J. Pharmacol. Exp. Ther. *125,* 237 (1959) which employs as nociceptive agent a 0.02% hydroalcoholic solution of 2-phenyl 1-4 benzoquinone, was used. 0.2 ml of this solution was administered by the i.p. route at a constant temperature of 37°C. The administration of this substance produced in the animals abdominal writhes which were easy to count. The animals used were female Swiss mice of weight 20 ± 2 g. The products to be studied, i.e. the compounds of the invention, phenylpiperazinoethanols of formula II and 2-(4-isobutylphenyl) propionic acid, were administered to the animals by the oral route one hour before the administration of the nociceptive agent. The value of $ED_{50}$ was calculated according to the method of Litchfield and Wilcoxon J. Pharmacol Exp. Ther. *99,* 96 (1949).

B. Acute toxicity ($LD_{50}$):

This test was carried out by administering the compounds of general formula I, the reference compounds phenylpiperazinoethanols of formula II and 2-(4-isobutylphenyl) propionic acid suspended in Tween 80 by gastric sondage at different dose levels. The doses administered were within those that do not produce any death in the animals $LD_0$, and those killing all the animals $LD_{100}$ during a 7-day observation period. The calculation of $LD_{50}$ was made according to Litchfield and Wilcoxon, and when this method could not be used, the method described by Reed and Muench Am. J. Hyg. *27,* 493 (1938) was employed.

The results of these studies together with the ratio between $LD_{50}$ and $ED_{50}$, which gives an idea of the therapeutic margin of each product are shown in tables 1 and 2.

The invention is further described in the following examples.

Example 1

2-(4-isobutylphenyl)-propionic acid chloride

To a 250 ml flask provided with a magnetic stirrer containing 20.6 g (0.1 mol) of 2-(4-isobutylphenyl)-propionic acid was added 11 ml (0.15 mol) of thionyl chloride and 1 ml of pyridine. The mixture was left at room temperature for a period of 3 hours, after which it was evaporated under vacuum. 100 ml of toluene were then added and the mixture evaporated again. The residue obtained was used without further purification. In this manner 19.9 g (85%) of 2-(4-isobutylphenyl) propionic acid chloride were obtained.

Example 2

2-[4-(3-chlorophenyl)-1-piperazinyl] ethanol

24.2 ml of 2-chloroethanol were added slowly from a dropping funnel to a mixture of 46.8 g of 1-(3-chlorophenyl) piperazine and 38.2 g of sodium carbonate in 300 ml of ethanol in a flask provided with mechanical stirrer and reflux condenser. The mixture was heated at reflux for 30 hours and then was filtered and washed with 100 ml of chloroform. The solvent was distilled off to dryness and the residue recrystallized from ethyl acetate to yield 52.3 g (91%) of a solid with a melting point of 98—100°C.

IR (Nujol®) ν $cm^{-1}$, 1590, 1550, 1460, 1050, 780.

TABLE 1

| Compounds of general formula I | $LD_{50}$ mg/kg p.o. | $ED_{50}$ mg/kg p.o. | $\frac{LD_{50}}{ED_{50}}$ |
|---|---|---|---|
| $R_2$ = H | 2,060 | 10.9 | 190 |
| $R_2$ = Cl | 1,930 | 6.75 | 286 |
| $R_2$ = $CF_3$ | 2,180 | 7.08 | 310 |
| 2(4-isobutylphenyl) propionic acid | 1,050 | 4.21 | 240 |

TABLE 2

| Compounds of general formula II III | $LD_{50}$ mg/kg p.o. | $ED_{50}$ mg/kg p.o. | $\frac{LD_{50}}{ED_{50}}$ |
|---|---|---|---|
| $R_2$ = H | 388 | 8.8 | 44 |
| $R_2$ = Cl | 408 | 4.11 | 100 |
| $R_2$ = $CF_3$ | 384 | 3.13 | 122 |

Example 3

2-[4-[3(trifluoromethyl)phenyl]-1-piperazinyl] ethanol

This compound was obtained in a similar manner to compound of example 2 by reacting 46 g of 1-[3(trifluoromethyl)phenyl]-piperazine with 32.1 g of sodium carbonate and 20 ml of 2-chloroethanol.

43.3 g (80%) of an oil were obtained.

IR (pure oil) ν $cm^{-1}$: 3400, 1590, 1570, 1460, 1050 and 750.

Example 4

2-(4-phenyl-1-piperazinyl) ethanol

This compound was also obtained in a similar manner to compound of example 2 by treating 48.6 g of 1-phenylpiperazine with 47 g of sodium bicarbonate and 30 ml of 2-chloroethanol.

There was obtained 47.7 g (72%) of a solid melting at 84—85°C.

IR (Nujol®) ν $cm^{-1}$: 1600, 1580, 1460, 1380, 1050, 760.

Example 5

2-[4-[3(trifluoromethyl)phenyl]-1-piperazinyl] ethyl 2-(4-isobutylphenyl) propionate, Hydrochloride

In 1 l flask 27 g (0.12 mol) of 2-(4-isobutylphenyl)-propionic acid chloride dissolved in 100 ml of toluene was added dropwise to a mixture of 27.4 g (0.1 mol) of 2-[4-[3(trifluoromethyl)phenyl]-1-piperazinyl] ethanol in 400 ml of toluene and 12.7 g of sodium carbonate. The temperature was maintained below 5°C. Once the addition has been completed, the mixture was stirred overnight. Then it was filtered and the filtrate was washed with water. The organic layer was dried over $MgSO_4$, filtered again and the filtrate was

evaporated to dryness. The residue obtained was dissolved in isopropanol, and 50% HCl was added to the precipitate the hydrochloride. In this manner were obtained 35 g (72%) of a white solid melting 174—176°C with a single spot in TLC (Rf = 0.67, n-butanol 9/acetic acid 1/water 3). Its elemental analysis gave:

| | %C | %H | %N | %Cl |
|---|---|---|---|---|
| Calculated for $C_{26}H_{34}Cl_3$ | 62.59 | 6.82 | 5.62 | 7.12 |
| Found | 62.93 | 7.16 | 6.02 | 7.00 |

I.R. (Nujol® v $cm^{-1}$: 1725 and 1175 (ester).

Example 6

2-[4-(3-chlorophenyl)-1-piperazinyl] ethyl 2(4-isobutylphenyl) proprionate, Hydrochloride

This compound was obtained in a similar manner to the compound of example 5 by reacting at low temperatures 24.05 g (0.1 mol) of 2-[4-(3 chlorophenyl)-1-piperazinyl] ethanol in toluene with 27.00 g (0.12 mol) of the acid chloride. The hydrochloride was isolated by bubbling hydrogen chloride into a solution of the base in isopropanol. 30 g (65%) of colorless crystals melting 165—167°C, were obtained.

| | %C | %H | %N | %Cl |
|---|---|---|---|---|
| Calculated for $C_{25}H_{34}Cl_2N_2O_2$ | 64.51 | 7.31 | 6.02 | 15.25 |
| Found | 64.41 | 7.43 | 6.20 | 15.59 |

IR (Nujol®) v $cm^{-1}$: 1730 and 1190 (ester).

Example 7

2-(4-phenyl 1-piperazinyl) ethyl (2-(4-isobutylphenyl) propionate, Hydrochloride

The above compound was obtained as in example 5 by reacting 20.6 g (0.1 ml) of 2-(4-phenyl 1-piperazinyl) ethanol dissolved in toluene with 27 g (0.1 mol) of 2 (4-isobutylphenyl) propionyl chloride, and obtaining the hydrochloride as described above. The hydrochloride obtained, 33 g (75%), melted at 153—155°C and its elemental analysis and IR spectrum were as follows:

| | %C | %H | %N | %Cl |
|---|---|---|---|---|
| Calculated for $C_{25}H_{35}ClN_2O_2$ | 69.69 | 8.13 | 6.50 | 8.24 |
| Found | 69.61 | 8.40 | 6.78 | 8.28 |

IR (Nujol®) v $cm^{-1}$: 1725 and 1180 (ester).

Example 8

The compounds of the invention may be provided in unit dosage forms as follows:

| | | |
|---|---|---|
| (a) | HARD GELATIN CAPSULES | |
| | Compound of the invention | 300 mg |
| | Mannitol | 200 mg |
| | Talc | 10.5 mg |
| | Magnesium stearate | 3.5 mg |
| (b) | TABLETS | |
| | Compound of the invention | 500 mg |
| | Microcrystalline cellulose | 115 mg |
| | Monocalcium phosphate. $2H_2O$ | 200 mg |
| | Magnesium stearate | 2.5 mg |
| (c) | SUPPOSITORIES | |
| | Compound of the invention | 700 mg |
| | Microcrystalline cellulose | 800 mg |
| | Triglycerides of fatty acids | 2,500 mg |
| (d) | INJECTABLE SOLUTION | |
| | Compound of general formula I | 100 mg |
| | EDTA $Na_2Ca$ | 0.025 mg |
| | Butyl hydroxyanisole | 0.1 mg |
| | Monosodium phosphate | 1.5 mg |
| | Hydroalcoholic vehicle s.q. | 10 ml |

**Claims**

1. Compound 2(4-phenyl-1-piperazinyl)ethyl 2(4-isobutylphenyl) propionate and its non-toxic physiologically acceptable salts.

2. Compound 2[4-(3-chlorophenyl)-1-piperazinyl)ethyl 2(4-isobutylphenyl) propionate and its non-toxic physiologically acceptable salts.

3. Compound 2[4-[3(trifluoromethyl)phenyl]-1-piperazinyl)ethyl 2(4-isobutylphenyl) propionate and its non-toxic physiologically acceptable salts.

4. A process for preparing a compound according to claims 1, 2 or 3, which comprises esterifying a phenylpiperazine ethanol derivative of general formula:

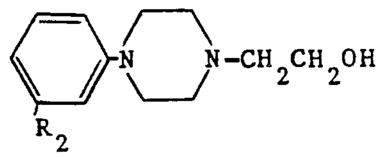

II

wherein $R_2$ represents a hydrogen or chlorine atom or a trifluoromethyl group, with an acid chloride of general formula:

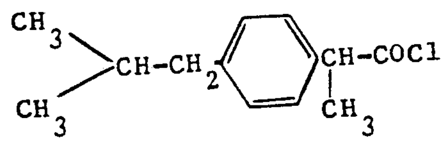

III

at low temperature and in presence of a base, and isolating the ester in the form of an organic or inorganic salt.

5. A process for preparing a compound according to claims 1, 2, or 3, which comprises esterifying 2-chloroethanol with 2(4-isobutylphenyl)-propionyl chloride to give a compound of formula:

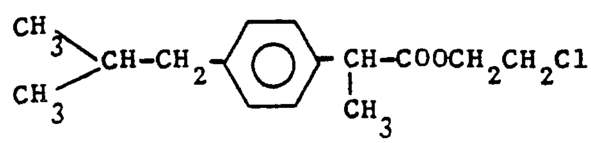

IV

and substituting the chlorine atom thereof with a phenylpiperazine of formula:

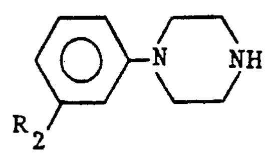

V

(wherein $R_2$ is a previously defined) by reaction in a basic medium.

6. Therapeutic compositions for oral, rectal or parenteral administration, which comprise a compound of claims 1 to 3, as well as non-toxic organic or inorganic salt thereof in association with pharmaceutically acceptable diluents or carriers.

**Patentansprüche**

1. 2(4-Phenyl-1-piperazinyl) ethyl 2(4-isobutylphenyl) Propionat-Verbindung und ihre nichtgiftigen physiologisch einwandfreien Salze.

2. 2-[4-(3-Chlorphenyl)-1-piperazinyl) ethyl 2(4-isobutylphenyl) Propionat-Verbindung und ihre nichtgiftigen physiologisch einwandfreien Salze.

3. 2(4-(3(Trifluormethyl)phenyl-1-piperazinyl) ethyl 2-(4-isobutylphenyl) Propionat-Verbindung und ihre nichtgiftigen physiologisch einwandfreien Salze.

4. Verfahren zur Herstellung einer Verbindung nach Anspruch 1, 2, oder 3, dadurch gekennzeichnet, daß ein Phenylpiperazinethanolderivat der allgemeinen Formel:

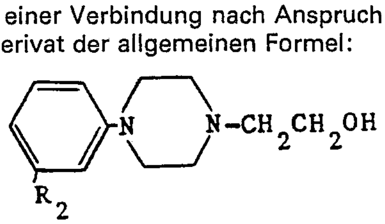

II

wobei $R_2$ ein Wasserstoff- oder Chloratom oder eine Trifluoromethylgruppe mit einer Chloridsäure der allgemeinen Formel:

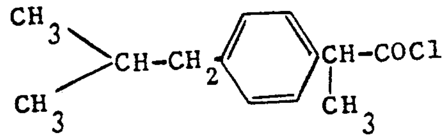

III

darstellt, bei niedrigen Temperaturen und in Gegenwart einer Base verestert wird, und daß der Ester in Form eines organischen oder anorganischen Salzes isoliert wird.

5. Verfahren zur Herstellung eine Verbindung nach Anspruch 1, 2 oder 3, dadurch gekennzeichnet, daß 2-Chlorethanol mit 2 (4-isobutylphenyl)-Propionylchlorid zur Bildung einer Verbindung der Formel:

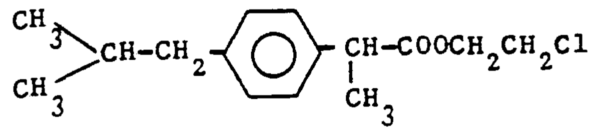

IV

verestert wird, und daß das Chloratom daraus mit einem Phenylpiperazin der Formel:

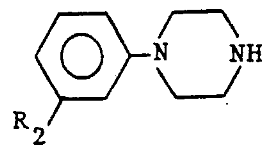

V

durch Reaktion in einer basischen Umgebung ersetzt wird, wobei $R_2$ vorausgehend definiert ist.

6. Therapeutische Zusammensetzungen zur oralen, rectalen oder parenteralen Anwendung, welche eine Verbindung nach den Ansprüchen 1 bis 3 sowie nicht giftige organische oder anorganische Salze in Verbindung mit pharmazeutisch einwandfreien Verdünnungsmitteln oder Carriern aufweist.

**Revendications**

1. Composé 2-(4-isobutylphényl)propionate de 2-(4-phénylpipérazinyl-1)éthyle et ses sels non toxiques physiologiquement acceptables.

2. Composé 2-(4-isobutylphényl)propionate de 2-[4-(3-chlorophényl)pipérazinyl-1]éthyle et ses sels non toxiques physiologiquement acceptables.

3. Composé 2-(4-isobutylphényl)propionate de 2-[4-(3-trifluorométhyl)phenyl]pipérazinyl-1-éthyle] et ses sels non toxiques physiologiquement acceptables.

4. Procédé pour préparer un composé selon les revendications 1, 2 ou 3 qui comprend l'estérification d'un dérivé de phényl pipérazinyléthanol de formule générale:

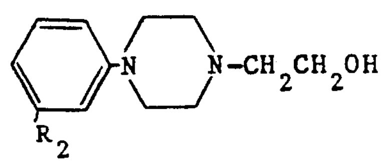

II

dans laquelle $R_2$ représente un hydrogène ou un atome de Chlore ou un groupe trifluorométhyle avec un chlorure d'acide de formule générale:

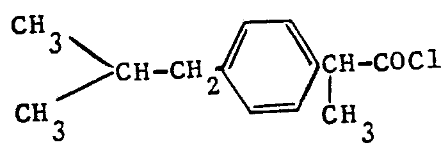

III

à basse température et en présence d'une base et l'isolation de l'ester sous la forme d'un sel organique ou minéral.

5. Procédé pour préparer un composé selon la revendication 1, 2 ou 3 qui comprend l'estérification du 2-chloroéthanol par le chlorure de 2-(4-isobutylphényl)propionyle pour donner un composé de formule:

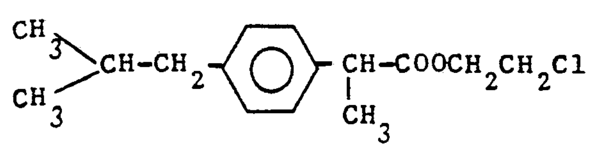

IV

et la substitution de l'atome de chlore de celui-ci par la phénylpipérazine de formule:

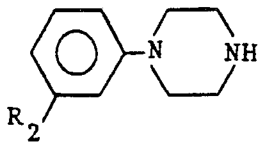

V

(dans laquelle $R_2$ est défini comme précédemment), par réaction dans un milieu basique.

6. Compositions thérapeutiques pour administration orale, rectale ou parentérale qui comprennent un composé des revendications 1 à 3 ainsi que les sels organiques un minéraux non toxiques de ceux-ci en association avec des diluants ou des supports pharmaceutiquement acceptables.